# EUROPEAN PATENT APPLICATION

(11) **EP 4 410 239 A1**
(43) Date of publication of application: **07.08.2024**
(21) Application number: 22875836.3
(22) Date of filing: 14.09.2022
(51) Int. Cl.: A61F 2/16

(54) **INTRAOCULAR LENS INSERTION TOOL**

(30) Priority: 30.09.2021 JP 2021161856
(71) Applicant: HOYA CORPORATION, Shinjuku-ku Tokyo 160-8347 (JP)
(72) Inventor: KUDO Kazunori, Tokyo 160-8347 (JP)
(74) Representative: MERH-IP Matias Erny Reichl Hoffmann Patentanwälte PartG mbB
(86) International application number: PCT/JP2022/034398
(87) International publication number: WO 2023/053965

(57) **Abstract**

There is provided an intraocular lens injector 1 for injecting into an eye an intraocular lens 4 having an optical portion 4a and a pair of support portions 4b, comprising: an injector main body 5 having a lens housing portion 11 for housing the intraocular lens 4, a slider 6 movably arranged along an injection direction of the intraocular lens 4, and a rod 10 movably arranged along the injection direction, separately from the slider 6 or in connection with the slider 6; the intraocular lens injector having, as a range of motion of the slider and the rod: a first range of motion S1 where a rear support portion 4b of the pair of the intraocular lens 4 in the lens housing portion 11 is tucked by a movement of the rod 10, and a second range of motion S2 where the intraocular lens is moved from the lens housing portion 11 to a predetermined position on the forward side of the injection direction by interlocking the slider 6 and the rod 10.

## Description

### Technical Field

The present invention relates to an intraocular lens injector.

### Description of Related Art

In cataract surgery, after removing a cloudy crystalline lens, an intraocular lens is injected into a lens capsule to replace the crystalline lens, and an intraocular lens injector is used to inject the intraocular lens. The concept of an intraocular lens injector equipped with a slider mechanism configured such that an intraocular lens that has been placed in the injector in advance is moved by a slider, folded into a small size, and then folded into a smaller size by a rod before being ejected from the injector, is known (for example, see Patent document 1).

### Prior art document

### Patent document

[Patent Document 1] Patent Publication No. 5413918

### Summary of the Invention

### Problem to be solved by the Invention

Folding the intraocular lens with the intraocular lens injector includes tucking a rear support portion of the intraocular lens. In the intraocular lens injector equipped with a slider mechanism described above, the slider simultaneously performs tucking of the rear support portion and folding of the intraocular lens. Therefore, it was necessary to temporarily suppress the folding of the intraocular lens using a folding control unit formed at a tip of the slider, to prevent interference between the tacked rear support portion and the folded intraocular lens. When the rod pushes the intraocular lens that is restrained and not properly folded, there is a risk of abrupt or uncontrolled ejection of the intraocular lens.

The present invention provides a technique for a smooth and controllable ejection of an intraocular lens from an intraocular lens injector equipped with a slider mechanism.

### Means for solving the Problem

First aspect of the present invention provides an intraocular lens injector for injecting into an eye an intraocular lens having an optical portion and a pair of support portions extending from the optical portion, comprising:
an injector main body having a lens housing portion for housing the intraocular lens to be injected,
a slider movably arranged along an injection direction of the intraocular lens which pushes out the intraocular lens housed in the lens housing portion, and
a stick-like rod movably arranged along the injection direction, separate from the slider or in connection with the slider;
the intraocular lens injector having, as a range of motion of the slider and the rod:
   a first range of motion where a rear support portion of the pair of support portions in the intraocular lens in the lens housing portion is tucked by a movement of the rod, and
   a second range of motion where the intraocular lens is moved from the lens housing portion to a predetermined position on the forward side of the injection direction by interlocking the slider and the rod while the rear support portion of the lens is being tacked.

A second aspect of the present invention provides the intraocular lens injector according to the first aspect,
wherein the intraocular lens injector has, as a range of motion of the slider and the rod, a third range of motion where the intraocular lens at the predetermined position is pushed out of the injector main body by moving the rod which is out of connection with the slider.

A third aspect of the present invention provides the intraocular lens injector according to the first or second aspect,
wherein the slider is configured to make contact with the intraocular lens at a plurality of points.

A fourth aspect of the present invention provides the intraocular lens injector according to any one of the first to third aspects,
wherein the slider and the rod are configured so that the movement of the rod is guided by the sliders that sandwich the rod.

A fifth aspect of the present invention provides the intraocular lens injector according to any one of the first to fourth aspects,
further comprising a slider tab movably arranged along the injection direction,
wherein the slider tab is configured to be in connection with the rod to cause the rod to move in the first range of motion, and to be in connection with the slider and the rod to cause the slider and the rod to move in the second range of motion. Advantage of the Invention

According to the present invention, in an intraocular lens injector equipped with a slider mechanism, the intraocular lens can be stably ejected.

### Brief description of the drawings

FIG. 1 is a perspective view illustrating an example of a configuration of an intraocular lens injector according to an embodiment of the present invention.
FIG. 2 is an explanatory view illustrating a specific example of a risk that may occur in an intraocular lens injector equipped with a slider mechanism.
FIG. 3 is an explanatory view (part 1) illustrating an example of the configuration of a main part of an intraocular lens injector according to an embodiment of the present invention.
FIG. 4 is an explanatory view (part 2) illustrating an example of the configuration of a main part of the intraocular lens injector according to an embodiment of the present invention.
FIG. 5 is an explanatory view (Part 1) illustrating an example of a processing operation in the intraocular lens injector according to an embodiment of the present invention.
FIG. 6 is an explanatory view (part 2) illustrating an example of a processing operation in the intraocular lens injector according to an embodiment of the present invention.

### Detailed description of the Invention

Embodiments of the present invention will be described below based on the drawings.

### (1) Basic configuration of an intraocular lens injector

First, a basic configuration of an intraocular lens injector will be explained.

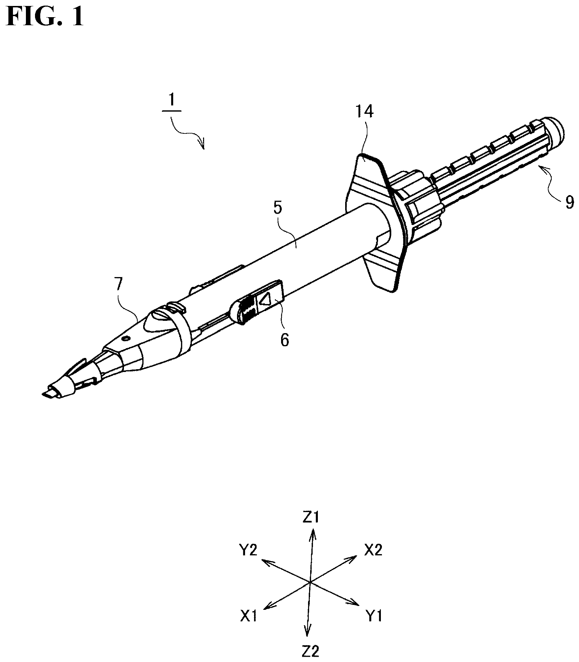

FIG. 1 is a perspective view illustrating an example of a configuration of the intraocular lens injector.

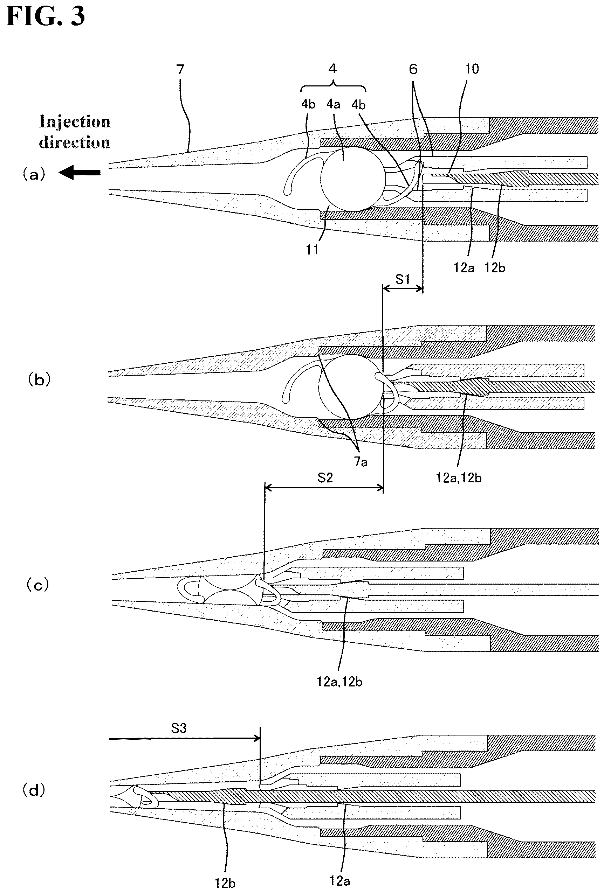

The intraocular lens injector 1 is used when injecting an intraocular lens 4 (not illustrated in FIG. 1) into an eye. The intraocular lens 4 comprises a soft material such as silicone elastomer or soft acrylic, and has a circular optical portion 4a that performs an optical function, and a pair of support portions 4b extending from an outer periphery of the optical portion 4a (for example, see FIG. 3 below). Since the intraocular lens 4 is well known, detailed explanation thereof will be omitted here.

In order to inject the intraocular lens 4 into an eye, as illustrated in FIG. 1, the intraocular lens injector 1 is roughly divided into an injector main body 5, a slider 6, a nozzle portion 7, a plunger 9, and a rod 10 connected to the plunger 9 (not illustrated in FIG. 1). Each of these components is preferably formed of a resin molded product.

The injector main body 5 is formed into a hollow tubular shape, and a nozzle portion 7 is connected to one end of the tube. The other end of the tube is provided with a flange 14 and an opening through which the plunger 9 and rod 10 can be inserted into the tube. A lens housing portion 11 (not illustrated in FIG. 1) that houses the intraocular lens 4 to be injected is provided within the tube of the injector main body 5.

The slider 6 is attached to the injector main body 5 and is arranged to be movable along an axial direction of the injector main body 5 (X-axis direction in FIG. 1). The axial direction of the injector main body 5 matches an injection direction when injecting the intraocular lens 4 housed in the injector main body 5 into an eye. Below, for explaining the injection direction of the intraocular lens 4, one of the X-axis directions is defined as X1 direction, and the other is defined as X2 direction, with the X1 direction being a front side (tip side) in the injection direction, with the X2 direction being a rear side (rear end side) in the injection direction. That is, the slider 6 is arranged movably along the injection direction (X-axis direction) of the intraocular lens 4. Thereby, the intraocular lens 4 housed in the lens housing portion 11 is pushed forward in the injection direction (X1 direction).

The nozzle portion 7 is for folding the intraocular lens 4 housed in the injector main body 5 into a small size and guiding it into an eye when the intraocular lens 4 is injected into the eye. For this purpose, the nozzle portion 7 has a lens outlet on a tip side, and a cross-sectional area of an internal space is configured to gradually become narrower from the side of the injector main body 5 toward the lens outlet.

The plunger 9 is arranged coaxially with the injector main body 5 and is arranged movably along the injection direction of the intraocular lens 4 (X-axis direction in FIG. 1). A stick-like rod 10 extending along the axial direction within the tube of the injection main body 5 is connected to the plunger 9. Accordingly, when the plunger 9 moves, the rod 10 arranged within the tube of the injector main body 5 also moves along the injection direction of the intraocular lens 4. That is, the rod 10 connected to the plunger 9 is also arranged movably along the injection direction of the intraocular lens 4. The plunger 9 and rod 10 move along the injection direction of the intraocular lens 4 in the same way as the slider 6, but the movement is performed separately from the slider 6 or in connection with the slider 6.

In the intraocular lens injector 1 configured as described above, when a user of the intraocular lens injector 1 (for example, a doctor performing cataract surgery) moves the slider 6, the intraocular lens 4 housed in the lens housing portion 11 within the injector main body 5 moves to a predetermined position on a front side in the injection direction. Thereby, the intraocular lens 4 is folded into a small size. Then, when the user moves the plunger 9, the rod 10 connected to the plunger 9 folds the intraocular lens 4 at a predetermined position into a further smaller size and pushes it outward. Thereby, the intraocular lens 4 is ejected from the lens outlet of the nozzle portion 7.

That is, the intraocular lens injector 1 is equipped with a slider mechanism of folding the intraocular lens 4 using the slider 6.

### (2) Risk in the slider mechanism

Here, the risk that may occur in an intraocular lens injector equipped with a slider mechanism will be briefly explained.

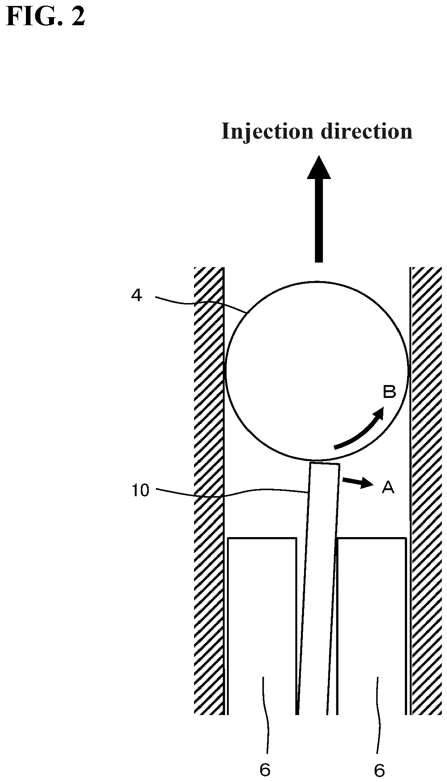

FIG. 2 is an explanatory view illustrating a specific example of the risk that may occur in the intraocular lens injector equipped with a slider mechanism.

In the intraocular lens injector equipped with a slider mechanism, the rod 10 pushes out the intraocular lens 4, thereby ejecting the intraocular lens 4 from the injector. In some cases, as illustrated in FIG. 2, if the rod 10 deviates from its normal path and contacts the lens at an angle due to inclination or bending of the rod 10 (see arrow A in the figure), this can create a force that rotates the lens, caused by the pushing of the rod 10 (see arrow B in the figure). That is, regarding the intraocular lens 4 ejected by the rod 10, there is a risk that a rotational force will be applied (hereinafter simply referred to as a "rotation risk").

Such a rotation risk leads to an unstable ejection of the intraocular lens 4. Specifically, when the rotational force is applied to the intraocular lens 4, the position of the support portions 4a and 4b of the intraocular lens 4 that is about to be ejected may be misaligned, and the user of the intraocular lens injector may not be able to perform the ejecting operation as expected, and may experience poor operability.

Accordingly, the rotation risk should be eliminated before it occurs. In order to eliminate the rotation risk, it is necessary to prevent the rod 10 from deviating from its normal path. Specifically, in order to prevent the rod 10 from deviating from its normal path, it is necessary to guide the forward movement of the rod 10 by the slider 6 while limiting the size of the gap between the slider 6 and the rod 10.

However, in the conventional intraocular lens injector disclosed in Patent document 1, the rotation risk cannot always be eliminated for the reasons described below.

In the intraocular lens injector equipped with a slider mechanism of folding the intraocular lens 4 into a small size and then ejecting it, the folding includes tacking of the support portion 4b on the rear side in the injection direction (hereinafter, simply referred to as a "rear support portion.") out of the pair of support portions 4b of the intraocular lens 4.

In the conventional intraocular lens injector, the slider 6 is used to tuck the rear support portion 4b. That is, the slider 6 not only has a function of moving the intraocular lens 4 within the injector, but also has a function of tacking the rear support portion 4b. For this reason, in the intraocular lens injector with a conventional configuration, an end shape of the slider 6 needs to be more complex and larger in order to perform multiple functions, which results in limiting a limiting amount of movement (stroke) toward the tip end of the nozzle portion 7 whose cross-sectional area gradually becomes narrower. When the stroke of the slider 6 is limited, the slider 6 cannot advance forward in the injection direction from there, and therefore the slider 6 cannot guide the rod 10 forward from there. Accordingly, this is not necessarily sufficient in terms of eliminating the rotation risk.

That is, the conventional intraocular lens injector involves the following problem: since the slider 6 has the function of tacking the rear support portion 4b, it is not necessarily possible to eliminate the rotation risk, and users of the intraocular lens injector experience poor operability.

This is a new finding obtained by the inventor of the present application. Based on this new finding, the inventor of the present application devised an intraocular lens injector 1 described in this embodiment in order to eliminate the above-described risk and to stably perform the ejection operation of the intraocular lens 4.

### (3) Configuration of a main part of intraocular lens injector

Next, a characteristic configuration of a main part of the intraocular lens injector 1 according to this embodiment will be explained.

FIG. 3 is an explanatory view (part 1) illustrating an example of the configuration of the main part of the intraocular lens injector 1 according to an embodiment of the present invention.

As illustrated in FIG. 3(a), the intraocular lens injector 1 houses the intraocular lens 4 in the lens housing portion 11 in the injector main body 5 so that the intraocular lens 4 can be ejected from the lens outlet of the nozzle portion 7. The intraocular lens 4 is ejected by moving the slider 6 and the rod 10 forward in the injection direction.

The slider 6 and the rod 10 may be moved separately or may be moved in connection each other. For this kind of behavior, the slider 6 and the rod 10 are each configured such that the movement of the rod 10 is guided by the sliders 6 that sandwich the rod 10. The slider 6 and the rod 10 are in connection each other using engaging portions 12a and 12b (see FIGS. 3(a) to 3(d)) provided therein respectively, and each connection can be canceled using elastic deformation of at least one of the slider 6 or the rod 10. Further, the connection state between the slider 6 and the rod 10 can be adjusted as desired (relative positions of the parts of the slider 6 and the rod 10 that touch the intraocular lens 4 in the advancing direction can be adjusted arbitrarily.), and by adjusting the connection state between the slider 6 and the rod 10, the degree of folding the tip of the rear support portion 4b in the advancing direction can be adjusted.

Then, in this embodiment, when moving the slider 6 and rod 10, a range of motion of the slider 6 and rod 10 is configured as illustrated in FIGS. 3(a) to 3(d). More specifically, the slider 6 and the rod 10 have a first range of motion S1, a second range of motion S2, and a third range of motion S3.

As illustrated in FIGS. 3(a) and 3(b), the first range of motion S1 is a range of motion in which the rod 10 mainly moves, and by this movement, this is a range of motion for tacking the rear support portion 4b of the pair of support portions 4b of the intraocular lens 4 in the lens housing portion 11. "Tacking" here refers to folding the rear support portion 4b toward a front side in the injection direction, and it does not matter whether or not the tip of the rear support portion 4b after folding is pushed between the folded optical portions 4a.

That is, in this embodiment, the rod 10 has a function of tacking the rear support portion 4b. Therefore, the slider 6 does not need to have a tacking function, and the end face shape can be prevented from becoming more complex and larger than in the case of the conventional configuration described above.

In the first range of motion S1, it is sufficient that the rod 10 is movable, and there is no need to move the slider 6. However, the slider 6 may be allowed to move as long as the rear support portion 4b can be tacked by the rod 10.

As illustrated in FIGS. 3(b) and 3(c), the second range of motion S2 is a range of motion in which the slider 6 and the rod 10 move together in connection each other, and this is a range of motion for moving the intraocular lens 4 with the rear support portion 4b tacked, from the lens housing portion 11 to a predetermined position on the front side in the injection direction. When the intraocular lens 4 is moved to a predetermined position, the optical portion 4a of the intraocular lens 4 is folded into a small size while the rear support portion 4b remains in a tacked state.

In the second range of motion S2, the slider 6 and the rod 10 are in connection each other to move the intraocular lens 4, and since the sliders 6 are configured to sandwich the rod 10, the slider 6 comes into contact with the intraocular lens 4 at a plurality of points (specifically, two points on both sides of the rod 10).

Accordingly, the rotation risk of the intraocular lens 4 can be suppressed to a lower level compared to the case where the intraocular lens 4 is pushed out using only the rod 10. At this time, when a protrusion 7a for temporarily stopping the optical portion 4a of the intraocular lens 4 is provided on a side wall that guides the movement of the intraocular lens 4, it becomes possible to adjust the position of the intraocular lens 4 at this point, which is required in order to reduce the rotation risk of the intraocular lens 4.

Further, in the second range of motion S2, the intraocular lens 4 is moved to a predetermined position on the front side in the injection direction, and since the end face shape can be prevented from becoming more complex and larger, it is possible to bring the predetermined position closer to the lens outlet of the nozzle portion 7, that is, to ensure a sufficient stroke of the slider 6 to the tip side of the nozzle portion 7, compared to the conventional configuration described above. The stroke of the slider 6 that is sufficiently ensured to the tip side allows the folding of the intraocular lens 4 to be promoted more than before, that is, a more stable state of the intraocular lens 4 can be obtained by bringing the intraocular lens 4 into closer contact with an inner surface of the nozzle portion 7 than before.

As illustrated in FIG. 3(d), the third range of motion S3 is a range of motion for moving the rod 10 that is not connected with the slider 6, and this is a range of motion for pushing out the intraocular lens 4 at a predetermined position from the lens outlet of the nozzle portion 7 (that is, outside the injector main body 5).

In the third range of motion S3, the intraocular lens 4 is pushed out by the rod 10, and in this case, by bringing a predetermined position, which is a position before being pushed out, closer to the lens outlet of the nozzle portion 7, a sufficient range for guiding the rod 10 by the slider 6 can be ensured. Accordingly, it is possible to effectively prevent the rod 10 from deviating from its normal path and suppress the rotation risk of the intraocular lens 4, compared to the case of the conventional configuration described above.

In the first range of motion S1, second range of motion S2, and third range of motion S3 as described above, the slider 6 and the rod 10 each operate in connection each other or separately. However, the mechanism for this purpose is not particularly limited, and may be any mechanism that is appropriately configured (designed) depending on the purpose. Specific operating modes of the slider 6 and the rod 10 will be described in detail later.

As described above, in the first range of motion S1, the movement of the rod 10 is used to tack the rear support portion 4b. For this purpose, the intraocular lens injector 1 has the following configuration.

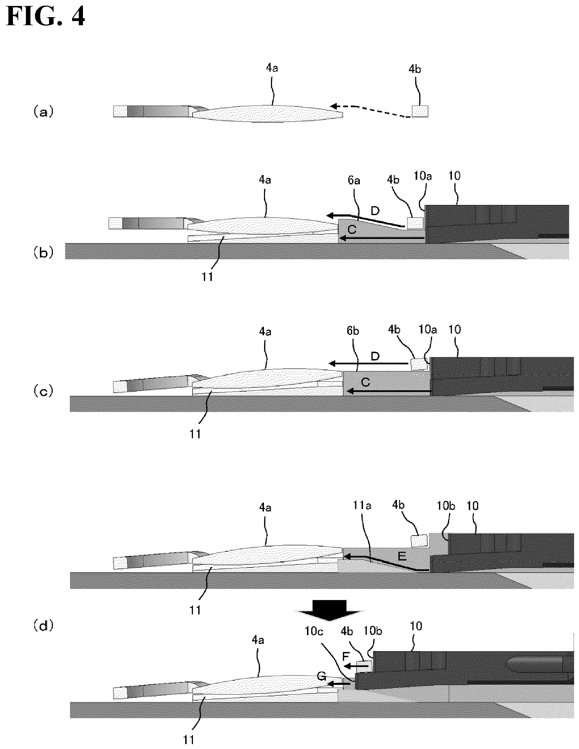

FIG. 4 is an explanatory view (part 2) illustrating an example of the configuration of a main part of the intraocular lens injector 1 according to this embodiment.

As illustrated in FIG. 4(a), when tacking the rear support portion 4b of the intraocular lens 4, a lower surface of the rear support portion 4b needs to be located at least higher than an upper end of an outer periphery of the optical portion 4a of the intraocular lens 4 (see dashed arrow in the figure).

For this purpose, for example, when the intraocular lens 4 is installed horizontally in the lens housing portion 11 (in other words, when the pair of support portions 4b are installed so as to be located at the same height) as illustrated in FIG. 4(b), the slider 6 is formed with a slope portion 6a for guiding the rear support portion 4b to the upper side of the optical portion 4a, and an end surface 10a of the rod 10 is formed in a shape capable of pressing the rear support portion 4b. Then, in the first range of motion S1, when the rod 10 moves forward in the direction of injection (inj ection direction) (see arrow C in the figure), the end surface 10a of the rod 10 presses against the rear support portion 4b of the intraocular lens 4. At this time, the rear support portion 4b is guided by the slope portion 6a and moves toward the upper side of the optical portion 4a (see arrow D in the figure). Then, when the rear support portion 4b reaches the upper side of the optical portion 4a, the rear support portion 4b is tucked. After tucking the rear support portion 4b, in the second range of motion S2, the optical portion 4a and the rear support portion 4b are pressed by the end surface 10a of the rod 10, and the optical portion 4a is also pressed by the slider 6. Although the case in which the slider 6 is formed with the slope portion 6a is exemplified here, the present invention is not limited thereto, and the slope portion 6a may be formed in the lens housing portion 11.

Further, for example, as illustrated in FIG. 4(c), when the intraocular lens 4 is installed obliquely at an angle so that a front side in the injection direction is lower in the lens housing portion 11, the slope portion 6a described above is not required, and a flat guide surface 6b may be formed on the slider 6. For such a configuration, in the first range of motion S1, when the rod 10 moves forward in the injection direction (see arrow C in the figure), the end surface 10a of the rod 10 presses against the rear support portion 4b of the intraocular lens 4. At this time, since the intraocular lens 4 is inclined, the rear support portion 4b is guided by the guide surface 6b and moves upward of the optical portion 4a (see arrow D in the figure). Then, when the rear support portion 4b reaches the upper side of the optical portion 4a, the rear support portion 4b is tucked. After the rear support portion 4b is tucked, the process is the same as that described above.

Further, for example, as illustrated in FIG. 4(d), when the intraocular lens 4 is installed obliquely in the lens housing portion 11, a slope portion 11a for guiding movement of the rod 10 may be formed in the lens housing portion 11. For such a configuration, a pressing surface 10b for pressing the rear support portion 4b of the intraocular lens 4 and a pressing surface 10c for pressing the optical portion 4a of the intraocular lens 4 are formed on the end edge of the rod 10. Then, in the first range of motion S1, when the rod 10 moves forward in the injection direction (see arrow E in the figure), the pressing surface 10b of the rod 10 presses the rear support portion 4b, and moves the rear support portion 4b toward the upper side of the optical portion 4a while being guided by the slope portion 11a (see arrow F in the figure), so that the rear support portion 4b is tucked. Thereafter, in the second range of motion S2, the optical portion 4a is pressed by the pressing surface 10c of the rod 10 (see arrow G in the figure) while the rear support portion 4b is pressed by the pressing surface 10b of the rod 10 (see arrow F in the figure), and further the optical portion 4a is also pressed by the slider 6. This causes the intraocular lens 4 to move forward in the injection direction.

### (4) Example of operation of intraocular lens injector

Next, as an example of the operation of the intraocular lens injector 1 having the above-described configuration, specific operating modes of the slider 6 and the rod 10 will be described.

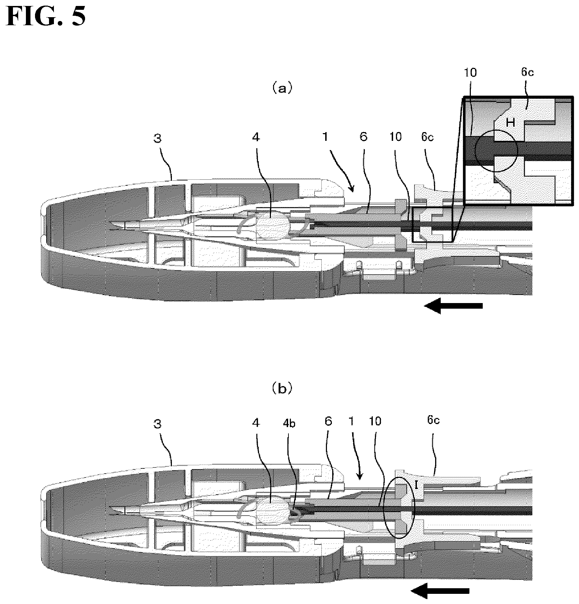

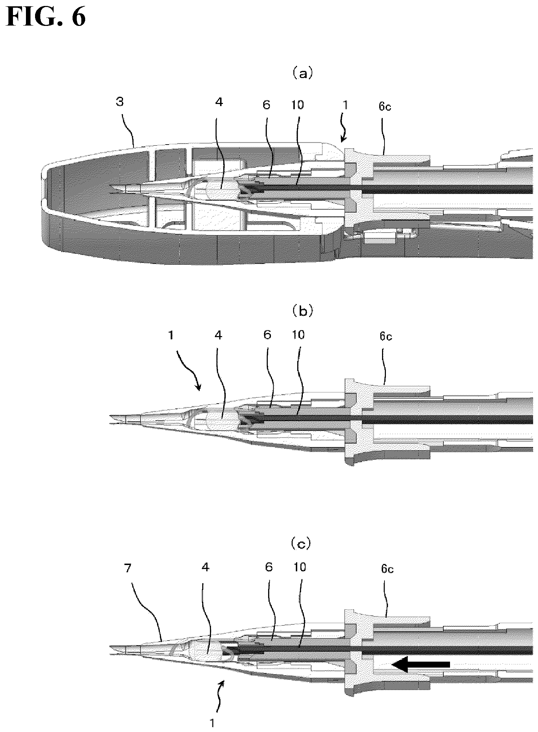

FIGS. 5 and 6 are explanatory views illustrating examples of the operation of the intraocular lens injector 1 according to this embodiment.

As illustrated in FIG. 5(a), when using the intraocular lens injector 1, it is assumed that the intraocular lens injector 1 is attached to a case 3, with the intraocular lens 4 preset in the lens housing portion 11. That is, after being shipped from a manufacturing factory, the intraocular lens injector 1 is attached to a special case 3 during transportation, preparation for surgery, etc. This prevents the preset intraocular lens 4 from being unnecessarily ejected or damaged.

Then, a user of the intraocular lens injector 1 first operates a slider tab 6c of the intraocular lens injector 1 manually moving the slider tab 6c forward in the injection direction of the intraocular lens 4 (see arrow in the figure). The slider tab 6c operated by the user constitutes a part of the slider 6, and is arranged to be movable along the injection direction of the intraocular lens 4 like the slider 6 and rod 10, and performs the operation in connection with the slider 6 and rod 10.

When the slider tab 6c moves forward in the injection direction, the slider tab 6c and the rod 10 are formed to engage with each other (see section H in the enlarged view). With this configuration, as the slider tab 6c moves, the rod 10 also moves forward in the injection direction. Although a specific manner of engagement between the slider tab 6c and the rod 10 is not limited, for example, as shown in section H in the figure, it is minimally necessary for them to engage with each other by providing a through hole in the center of the slider tab 6c in the Y direction, passing the rod 10 through this through hole, and making the X1 direction side of the rod 10 wider than the through hole. In this mode, when the slider tab 6c is moved forward in the X1 direction, the rod 10 and the slider tab 6c engage with each other, and the slider tab 6c and the rod 10 move forward together. The movement of rod 10 at this time corresponds to the movement in the first range of motion S1. That is, the slider tab 6c causes the rod 10 to move in the first range of motion S1, in connection with the rod 10.

Accordingly, in the first range of motion S1, as illustrated in FIG. 5(b), when the rod 10 moves, the preset rear support portion 4b of the intraocular lens 4 is pushed forward in the injection direction by the rod 10. That is, by moving the rod 10, the rear support portion 4b of the intraocular lens 4 is tucked.

When the movement of the first range of motion S1 is completed and the rear support portion 4b is in a tacked state, the slider tab 6c engages not only with the rod 10 but also with the slider 6 (see part I in the figure). Although this engagement mode is not limited, for example, as illustrated in part I in the figure, the shape of the slider tab 6c on the X1 direction side and the shape of the slider 6 on the X2 direction side may be shaped to fit into each other.

Thereafter, when the user continues to move the slider tab 6c, as illustrated in FIG. 6(a), both the slider 6 and the rod 10 move forward in the injection direction together with the movement of the slider tab 6c, because the slider tab 6c is engaged not only with the rod 10 but also with the slider 6. The movement of the slider 6 and the rod 10 at this time corresponds to the movement in the second range of motion S2. That is, the slider tab 6c causes the slider 6 and the rod 10 to move in the second range of motion S2, in connection with the slider 6 and the rod 10.

Accordingly, when the slider 6 and the rod 10 move in the second range of motion S2, the intraocular lens 4 with the rear support portion 4b tacked is pushed forward in the injection direction by both the slider 6 and the rod 10. At this time, the slider 6 contacts the intraocular lens 4 at a plurality of points (specifically, two points on both sides of the rod 10). Therefore, the risk of intraocular lens 4 rotation is lowered compared to a case where the intraocular lens 4 is pushed out using only the rod 10.

Then, the slider 6 and the rod 10 are moved until the intraocular lens 4 reaches a predetermined position on the front side in the injection direction. At this time, since the end face shape of the slider 6 can be prevented from becoming more complex and larger, it is possible to bring the predetermined position closer to the lens outlet of the nozzle portion 7, that is, to ensure a sufficient stroke of the slider 6 to the tip side of the nozzle portion 7, compared to the conventional configuration described above.

When the intraocular lens 4 reaches the predetermined position and completes the movement in the second range of motion S2, at that stage, the intraocular lens injector 1 is disengaged from the case 3 and can be removed from the case 3, as illustrated in FIG. 6(b).

Thereafter, the user of the intraocular lens injector 1 injects the intraocular lens 4 housed in the intraocular lens injector 1 into the eye of a subject (patient). Specifically, the user of the intraocular lens injector 1 manually operates the plunger 9 of the intraocular lens injector 1, as illustrated in FIG. 6(c), and causes the rod 10 connected to the plunger 9 to move forward in the injection direction of the intraocular lens 4 (see the arrow in the figure). The movement of the rod 10 at this time corresponds to the movement in the third range of motion S3. That is, the plunger 9 causes the connected rod 10 to move in the third range of motion S3. At this time, the rod 10 having been out of connection with the slider tab 6c, can move only the rod 10 forward in the injection direction (X1 direction).

Accordingly, when the rod 10 moves in the third range of motion S3, the intraocular lens 4 at a predetermined position is pushed forward in the injection direction by the rod 10, and is ejected to the outside from the lens outlet of the nozzle portion 7 when passing through the nozzle portion 7, while being folded into a small size.

At this time, the rod 10 that pushes out the intraocular lens 4 has been out of connection with the slider 6, and is guided forward in the injection direction by the slider 6. Therefore, by bringing the predetermined position, which is the position before being pushed out, closer to the lens outlet of the nozzle part 7, a sufficient guide range by the slider 6 can be ensured, and it is possible to effectively prevent the rod 10 from deviating from its normal path thereby lowering the risk of intraocular lens 4 rotation, compared to the case of the conventional configuration described above.

Through the processing operations described above, the intraocular lens 4, which is ejected from the lens outlet of the nozzle portion 7, is injected into the eye of the subject. That is, by injecting the tip of the nozzle portion 7 of the intraocular lens injector 1 into the eye and ejecting the intraocular lens 4 from the lens outlet of the nozzle portion 7 in this state, the user of the intraocular lens injector 1 achieves the injection of the intraocular lens 4 into the eye.

### (5) Effects of this embodiment

According to this embodiment, one or more of the following effects can be obtained.

(a) In this embodiment, the slider 6 and the rod 10 have at least the first range of motion S1 and the second range of motion S2. Then, in the first range of motion S1, the rod 10 moves to tack the rear support portion 4b, and in the second range of motion S2, the slider 6 and the rod 10 move in connection with the rod 10, to move the intraocular lens 4 to a predetermined position.

That is, since the rod 10 has a function of tacking the rear support portion 4b, the slider 6 does not have to have the tacking function, and compared to the conventional configuration described above, the end face shape can be prevented from becoming more complex and larger. Further, since the slider 6 and the rod 10 are in connection each other to move the intraocular lens 4 in the second range of motion S2, the risk of intraocular lens 4 rotation is lowered, compared to the case where the intraocular lens 4 is pushed out using only the rod 10. In addition, although the intraocular lens 4 is moved to a predetermined position on the front side in the injection direction in the second range of motion S2, the end face shape of the slider 6 can be prevented from becoming more complex and larger. Therefore, it is possible to bring the predetermined position closer to the lens outlet of the nozzle portion 7, that is, to ensure a sufficient stroke of the slider 6 to the tip side of the nozzle portion 7, compared to the conventional configuration described above.

Accordingly, in this embodiment, it becomes possible to eliminate the risk that the ejection operation of the intraocular lens 4 becomes unstable, and according to the intraocular lens injector 1 equipped with a slider mechanism, the intraocular lens 4 can be stably ejected.

(b) In this embodiment, the slider 6 and the rod 10 have the third range of motion S3 in addition to the first range of motion S1 and the second range of motion S2. Then, by moving the rod 10 having been out of connection with the slider 6 in the third range of motion S3, the intraocular lens 4 at a predetermined position is pushed out of the injector main body from the lens outlet of the nozzle portion 7.

Accordingly, in this embodiment, a sufficient range in which the rod 10 is guided by the slider 6 can be ensured. Therefore, it is possible to effectively prevent the rod 10 from deviating from its normal path and lower the risk of intraocular lens 4 rotation compared to the case of the conventional configuration described above.

(c) In this embodiment, the slider 6 is configured to come into contact with the intraocular lens 4 at a plurality of points. Accordingly, this is very effective in lowering the risk of intraocular lens 4 rotation when the slider 6 moves the intraocular lens 4.

(d) In this embodiment, the slider 6 and the rod 10 are configured to guide the movement of the rod 10 by the sliders 6 that sandwiches the rod 10. Accordingly, this is very effective in effectively preventing the rod 10 from deviating from its normal path and reducing the rotation risk of the intraocular lens 4.

(e) In this embodiment, the intraocular lens 4 includes a slider tab 6c that is movably arranged along the injection direction of the intraocular lens 4, and the slider tab 6c is configured to operate in connection with the slider 6 and the rod 10. That is, the slider tab 6c causes the rod 10 to move in the first range of motion S1 in connection with the rod 10, and causes the slider 6 and the rod 10 to move in the second range of motion S2 in connection with the slider 6 and the rod 10.

Accordingly, this embodiment enables the following situation: the user of the intraocular lens injector 1 causes the rod 10 to move in the first range of motion S1, and causes the slider 6 and rod 10 to move in the second range of motion S2, by operating (moving) the slider tab 6c. That is, for the user of the intraocular lens injector 1, this provides excellent operability, which is preferable for stably performing the operation of ejecting the intraocular lens 4.

### (6) Modified example, etc.

The embodiments of the present invention have been described above, but the disclosures described above indicate exemplary embodiments of the present invention. That is, the technical scope of the present invention is not limited to the above-described exemplary embodiments, and various changes can be made without departing from the claimed subject matter.

For example, the embodiment described above shows a case in which each component of the intraocular lens injector 1 is formed of a resin molded product. However, the present invention is not limited thereto, and each component of the intraocular lens injector 1 may be formed of a metal member such as stainless steel or titanium.

### Description of signs and numerals

1... Intraocular lens injector, 4... Intraocular lens, 4a... Optical portion, 4b... Support portion (rear support portion), 5... Injector main body, 6... Slider, 7... Nozzle portion, 9... Plunger, 10... Rod, 11... Lens housing portion, S1... First range of motion, S2... Second range of motion, S3... Third range of motion

## Claims

1. An intraocular lens injector for injecting into an eye an intraocular lens having an optical portion and a pair of support portions extending from the optical portion, comprising:
an injector main body having a lens housing portion for housing the intraocular lens to be injected,
a slider movably arranged along an injection direction of the intraocular lens which pushes out the intraocular lens housed in the lens housing portion, and
a stick-like rod movably arranged along the injection direction, separate from the slider or in connection with the slider;
the intraocular lens injector having, as a range of motion of the slider and the rod:
a first range of motion where a rear support portion of the pair of support portions in the intraocular lens in the lens housing portion is tucked by a movement of the rod, and
a second range of motion where the intraocular lens is moved from the lens housing portion to a predetermined position on the forward side of the injection direction by interlocking the slider and the rod while the rear support portion of the lens is being tacked.

2. The intraocular lens injector according to claim 1,
wherein the intraocular lens injector has, as a range of motion of the slider and the rod, a third range of motion where the intraocular lens at the predetermined position is pushed out of the injector main body by moving the rod which is out of connection with the slider.

3. The intraocular lens injector according to claim 1 or 2,
wherein the slider is configured to make contact with the intraocular lens at a plurality of points.

4. The intraocular lens injector according to any one of claims 1 to 3,
wherein the slider and the rod are configured so that the movement of the rod is guided by the sliders that sandwich the rod.

5. The intraocular lens injector according to any one of claims 1 to 4,
further comprising a slider tab movably arranged along the injection direction,
wherein the slider tab is configured to be in connection with the rod to cause the rod to move in the first range of motion, and to be in connection with the slider and the rod to cause the slider and the rod to move in the second range of motion.
